# EUROPEAN PATENT APPLICATION

(11) **EP 2 586 774 A1**
(43) Date of publication of application: **01.05.2013**
(21) Application number: 10853535.2
(22) Date of filing: 02.12.2010
(51) Int. Cl.: C07D 295/15, A61K 31/495, A61P 9/10

(54) **METHOD FOR PREPARATION OF RANOLAZINE**

(30) Priority: 25.06.2010 CN 201010211333
(71) Applicant: Shanghai Kuoikee Laboratories Co., Ltd, Shanghai 201203 (CN); Zhejiang Jianfeng Haizhou Pharmaceutical Co., Ltd, Linhai, Zhejiang 317016 (CN)
(72) Inventor: SHI, Mingfeng, Shanghai 201208 (CN); LI, Dan, Shanghai 201203 (CN); XU, Ling, Shanghai 201203 (CN)
(74) Representative: Clarenbach, Carl-Philipp
(86) International application number: PCT/CN2010/079368
(87) International publication number: WO 2011/160396

(57) **Abstract**

A process for the preparation of ranolazine comprises the step of condensing N-(2,6-dimethylphenyl)-1-piperazinyl acetamide with a compound of formula (I) to obtain ranolazine, in which X is chlorine or bromine. Ranolazine is prepared by condensing ring-opening halide which replaces epoxide in this process.

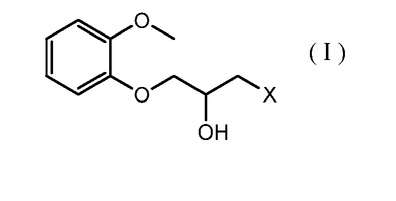

## Description

### Technical Field of the Invention

The present invention relates to the technical field of chemicals, and more particularly to a process for the preparation of an antianginal agent ranolazine.

### Background of the Invention

Ranolazine, chemically known as (±)-N-(2,6-dimethylplenyl)-4-[2-hydroxy-3-(2-methoxyphenoxy) propyl]-1-piperazineacetamide, is represented by the formula as given below.

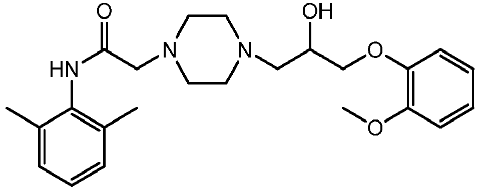

Ranolazine, a novel agent used to treat angina pectoris type coronary heart disease, was developed by American CV Therapeutica Company (now known as Gilead Sciences Company). Ranolazine has firstly been appeared on the market in US in 2006 and could be used to treat myocardial infarction, congestive heart disease, angina and arhythmia etc. The mechanism of action of ranolazine is to inhibit partial fatty acid oxidation, which changes fatty acid oxidation to glucose oxidation in heart, and thereby reduces the cardiac oxygen consumption. Ranolazine is the only antianginal agent without changing heart rate or blood pressure.

The processses for the preparation of ranolazine, which could be roughly divided into two types as shown in Fig. 1 and Fig. 2, were disclosed in International Application Publication No. WO 2010/025370, WO 2010/023687, WO 2009/153651, WO 2008/139492, WO 2008/047388, WO 2006/008753, Chinese patent No. CN101560196, CN101544617, CN1915982, the United States patent No. US2008312247, the publication China Pharmacist, 2007, 10(12), 1176-1177, Chinese Journal of Medicinal Chemistry, 2003, 13(5), 283-285, and Chinese Journal of Pharmaceuticals, 2004, 35(11): 641-642.

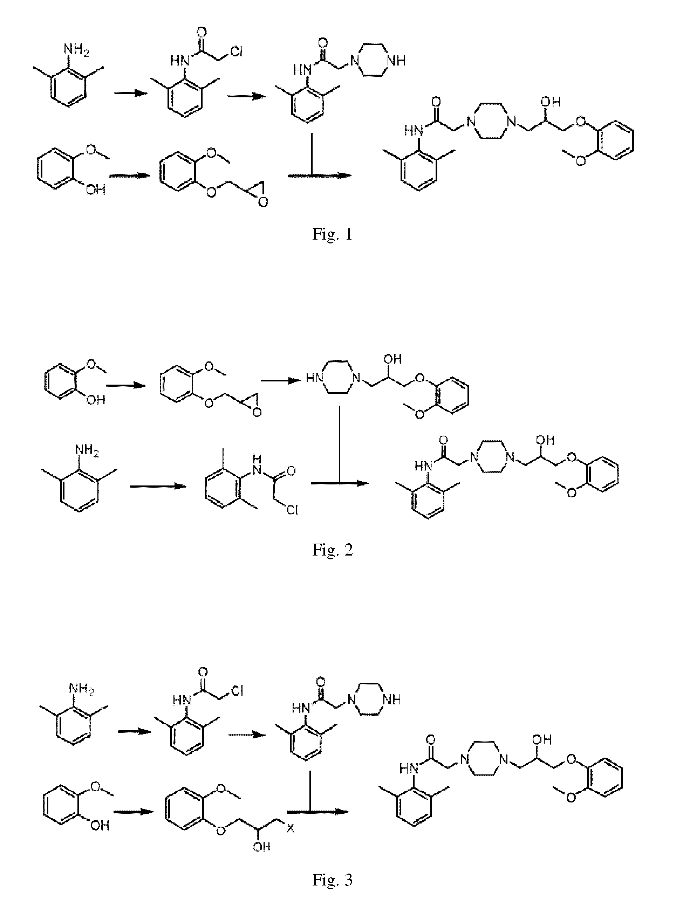

The process described in Fig. 1 (method 1) involves reacting [(2,6-dimethylphenyl)-carbamylmethyl]-peperazine with 1-(2-methoxyphenoxy)- 2,3-epoxypropane to obtain ranolazine, in which comprises the steps of :
a) condensing 2,6-xylidine with chloroacetyl chloride in the presence of base to get amide, which is further reacted with piperazine by a substitution reaction of N-monoalkylation to get N-(2,6-dimethylphenyl)-1-piperazineacetamide, and
b) condensing guaiacol with epoxy chloropropane to get 1-(2-methoxyphenoxy)-2,3-epoxypropane.

As the condensation is carried out in the alkaline environment, the epoxy ring becomes easy to open loop, and thus the products comprise mixtures of open-looped and looped form, thereby requiring rigorous separation conditions and being difficult to achieve the desired purity in the following reaction.

The process described in Fig. 2 (method 2) involves reacting 2-chloro-N-(2,6-dimethylphenyl)-acetamide with 1-(2-methoxyphenoxy)-3-(N- piperazine)-2-hydroxypropane to get ranolazine, in which comprises the steps of :
a) condensing 2,6-xylidine with chloroacetyl chloride in the presence of base to get 2-chloro-N-(2,6-dimethylphenyl)-acetamide, and
b) condensing guaiacol with epoxy chloropropane to get 1-(2-methoxyphenoxy)-2,3-epoxypropane, which is further reacted with piperazine to get 1-(2-methoxyphenoxy)-3-(N-piperazine)-2-hydroxypropane.

As the condensation is carried out in the alkaline environment, the epoxy ring becomes easy to open loop, and thus the products comprise mixtures of open-looped and looped form, thereby requiring rigorous separation conditions and being difficult to achieve the desired purity in the following reaction. The monosubstitution reaction of N-alkylation reacted with peperazine is further difficult to be controlled to produce the desired products.

Compared with method 2, method 1 could be easier to be industrialized as the quality of intermediates obtained by method 1 could be easier to be controlled and also the method 1 could be easier to be operated. But in the repeated experiments, it was found that it still had a lot of difficulties in realizing the industrialization by method 1 although it could be easier to be operated as there are mixtures including open-looped and looped products rather than single product produced when guaiacol (o-methoxyphenol) was reacted with epoxy chloropropane, so the operation of distillatory separation would still need very high temperature (above 250°C) and very low vacuum degree (5mm Hg) with the disadvantages of high energy consumption, high facilities investment and tedious operation. And in the following condensation reaction, there are a lot of products were produced during the reaction so as to make the quality of the products hard to be controlled.

### Abstract of the Invention

Through extensive and in-depth research, the present inventor amazedly found that ranolazaine with high purity could be easily obtained by condensing ring-opening halide which replaces epoxide of the prior art.

Accordingly the present invention provides a process for preparating ranolazine to make the quality of ranolazine easy to control so as to overcome the disadvantages of the prior art.

The process for the preparation of ranolazine provided by the present invention comprises a step of condensing N-(2,6-dimethylphenyl)-1-piperazinylacetamide with a compound of formula as given below to obtain ranolazine, in which X is chlorine or bromine.

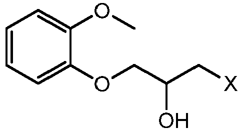

According to the present invention, the condensation is carried out in the presence of alcohol, toluene or mixtures thereof and heated under reflux in an alkaline environment.

According to the present invention, the mole ratio of N-(2,6-dimethylphenyl)-1 -piperazinylacetamide to the compound of formula as given below is 0.8~1.3:1.

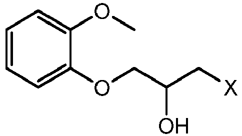

According to the present invention, the condensation reaction is carried out for 3~5 h.

According to the present invention, the alcohol is selected from methanol, ethanol, normal propyl alcohol and isopropyl alcohol. The alkaline environment is formed by sodium carbonate, sodium bicarbonate, potassium carbonate, potassium bicarbonate or mixtures thereof.

The present invention provided a process for preparating ranolazine with high purity easily by condensing ring-opening halide which replaces epoxide of the prior art. Compared to the epoxide, there is no three-membered ring with high-tensile strength in the molecular structure of ring-opening halide, which makes the compound more stable, lower boiling point, easier to separate to prepare substances with high purity, and thereby the following preparation of ranolazine by condensing becomes easier to be controlled and industrialized.

### Description of the Drawings

Fig. 1 is a synthetic map of ranolazine provided by the prior art.
Fig. 2 is another synthetic map of ranolazine provided by the prior art.
Fig. 3 is the synthetic map of ranolazine provided by the present invention.

### Detailed Description of the Invention

The present invention is further illustrated by the following particular embodiments. It should be understood that the following embodiments are only used to illustrate the present invention, but not to limit the scope of the present invention.

In the following embodiments, as shown in Fig. 3, on the one hand, 2,6-xylidine is used as a raw material to synthetize 2-chloro-N-(2,6-dimethylphenyl)-acetamide, which is further reacted with piperazine to get N-(2,6-dimethylphenyl)-1-piperazineacetamide, and on the other hand, guaiacol is used as a raw material to synthetize a compound of formula as given below, in which X is chlorine or bromine.

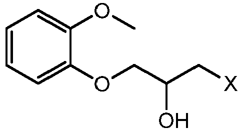

Finally, ranolazine is synthetized by condensing N-(2,6-dimethylphenyl)-1-piperazineacetamide with the above-mentioned compound.

### Example 1: Preparation of N-(2,6-dimethylphenyl)-1-piperazinylacetamide

### 1.1: Preparation of 2-chloro-N-(2,6-dimethylphenyl)-acetamide

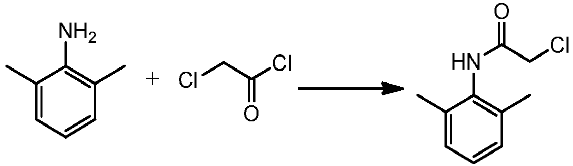

30.5 g (0.252 mol) of 2,6-xylidine, 100 ml of ethyl acetate, 26.5 g (0.25 mol) of sodium carbonate were successively added into a 250 ml of 3-neck flask and placed in an ice-water bath. 36.5 g (0.323 mol) of chloroacetyl chloride was dissolved in 50 ml of ethyl acetate and then the mixture was dropwise added into the 3-neck flask till completion. The ice-water bath was removed and the reaction was carried out for 3 h at the room temperature. The reaction product was slowly added 100 ml of water in an ice-water bath, stirred for 10 min and filtered. The filter cake as white needle solid was washed and dried under vacuum to get 46.3 g of 2-chloro-N-(2,6-dimethylphenyl)-acetamide having a yield of 93%.

### 1.2: Preparation of N-(2,6-dimethylphenyl)-1-piperazinylacetamide

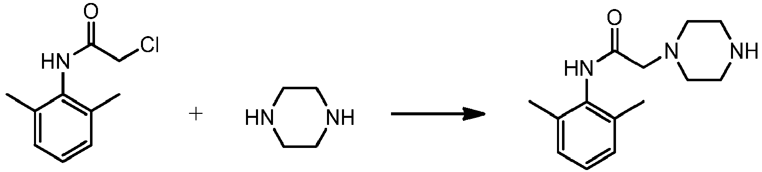

58.3 g (0.3 mol) of piperazine hexahydrate was dissolved in 230 ml of ethanol and 50.0 g (0.25 mol) of 2-chloro-N-(2,6-dimethylphenyl)-acetamide was subsquently added. The mixture was heated under reflux for 3 h till completion. The reaction product was cooled to room temperature and filtered. The filter was concentrated under reduced pressure and 80 ml of water was added. The mixture was extracted with dichloromethane and the organic layer was concentrated under vacuum at 60°C to get 39.4 g of N-(2,6-dimethylphenyl)-1-piperazinylacetamide having a yield of 63%. ¹HNMR (CDCl₃): 2.23~2.27,s,6H; 2.67,s,4H; 2.96~2.98,t,4H; 3.19~3.21,s,2H; 7.08~7.26,m,3H; 8.69,s,1H.

### Example 2: Preparation of ring-opening halide

### 2.1: Preparation of 1-chloro-3-(2-methoxyphenoxy)-2-propylalcohol

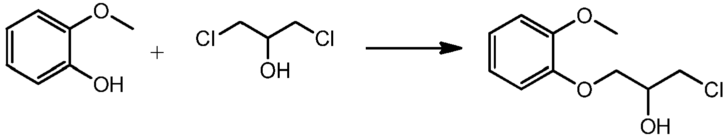

26 g (0.65 mol) of sodium hydroxide, 150 ml of water, 150 ml of ethanol, 62 g (0.5 g) of guaiacol were successively added into a reaction flask and 103 g (0.8 mol) of 1,3-dichloro-2-propylalcohol was slowly dropwise added till completion. The mixture was heated up to 45°C for 24 h. The reaction product was extracted three times with 150 ml of dichloromethane each and the organic layer was combined, dried with anhydrous magnesium chloride and distilled under reduced pressure. The fraction at 160°C and a pressure of 2 kp was collected to get 73.6 g of faint yellow liquid having a yield of 68%. ¹HNMR (CDCl₃): 3.44~3.46,d,1H; 3.69~3.78,dd,2H; 3.85,s,3H; 4.11~4.12,d,2H; 4.18~4.22,m,1H; 6.89~7.00,m,4H. The result confirmed that the yellow liquid was 1-chloro-3-(2- methoxyphenoxy)-2-propylalcohol.

### 2.2: Preparation of 1-bromo-3-(2-methoxyphenoxy)-2-propylalcohol

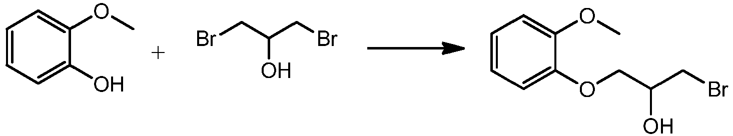

26 g (0.65 mol) of sodium hydroxide, 150 ml of water, 150 ml of ethanol, 62 g (0.5 g) of guaiacol were successively added into a reaction flask and 174.4 g (0.8 mol) of 1,3-dibromo-2-propylalcohol was slowly dropwise added till completion. The mixture was heated up to 45°C for 10 h. The reaction product was extracted three times with 150 ml of dichloromethane each and the organic layer was combined, dried with anhydrous magnesium chloride and distilled under reduced pressure. The fraction at 160°C and a pressure of 2 kp was collected to get 103 g of faint yellow liquid of 1-bromo-3-(2-methoxyphenoxy)-2-propylalcohol having a yield of 79%.

### Example 3: Preparation of ranolazine

### 3.1: 1-chloro-3-(2-methoxyphenoxy)-2-propylalcohol as a raw material

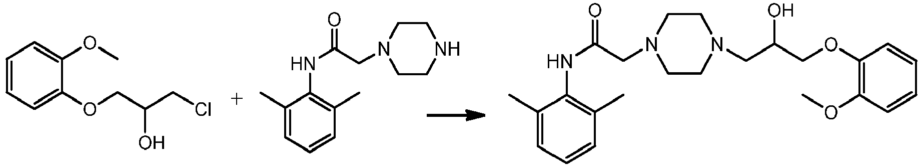

2.5 g (0.01 mol) of 1-chloro-3-(2-methoxyphenoxy)-2-propylalcohol, 3.1 g (0.012 mol) of N-(2,6-dimethylphenyl)-1-piperazinylacetamide, 4.1 g (0.03 mol) of potassium carbonate, 25 ml of methanol and 50 ml of toluene were successively added into a reaction flask and heated under reflux for 4.5 h till completion.

The fraction whose main ingredient was methanol was collected by atmospheric distillation at boiling point of 62-68°C and then filtrated. The filtrate was washed with 3N HCl to get 50 ml of liquid having a pH of 1-2 and further treated with 50 ml of saturated sodium carbonate solution to adjust pH to 9-10. The product was extracted three times with 20 ml of dichloromethane each and the lower organic phase was combined. After the dichloromethane was removed by distillation under reduced pressure and rotary evaporation, the yellow viscous liquid was obtained and then further dissolved in about 10 ml of methonal. The tetrahydrofuran was then dropwise added under reflux till turbidity. The product was slowly crystallized with cooling and filtrated to get 3.42 g of white solid having a yield of 80.1% by vacuum drying at 40°C.

¹HNMR (CDCl₃): 2.22,s,6H; 2.60~2.62,t,4H; 2.75,s,6H; 3.21,s,2H; 3.45,s,3H; 3.85,s,3H; 4.02~4.04,t,2H; 4.16,s,1H; 6.88~6.90,t,2H; 6.91~6.96,m,2H; 7.08~7.1,m,3H; 8.65,s,1H. The result confirmed that the compound obtained is ranolazine. Purity by HPLC (area normalization method): 99.1%.

### 3.2: 1-chloro-3-(2-methoxyphenoxy)-2-propylalcohol as raw material

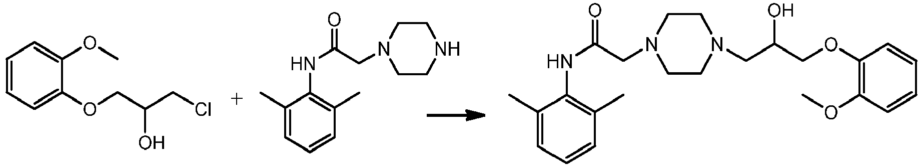

2.5 g (0.01 mol) of 1-chloro-3-(2-methoxyphenoxy)-2-propylalcohol, 2.8 g (0.011 mol) of N-(2,6-dimethylphenyl)-1-piperazinylacetamide, 3.0 g (0.03 mol) of potassium bicarbonate and 65 ml of toluene were successively added into a reaction flask and heated up to 80-85°C for 4.5 h till completion.

After cooling and filtering, the filtrate was washed with 3N HCl to get liquid having a pH of 1-2 and further treated with 50 ml of saturated sodium carbonate solution to adjust pH to 9-10. The product was extracted three times with 20 ml of dichloromethane each and the lower organic phase was combined. After the dichloromethane was removed by distillation under reduced pressure and rotary evaporation, the yellow viscous liquid was obtained and then further dissolved in about 10 ml of methonal. The tetrahydrofuran was then dropwise added under reflux till turbidity. The product was slowly crystallized with cooling and filtrated to get 2.62 g of white solid having a yield of 61.4% by vacuum drying at 40°C.

The result of ¹HNMR (CDCl₃) confirmed that the compound obtained is ranolazine. Purity by HPLC (area normalization method): 98.6%.

### 3.3: 1-chloro-3-(2-methoxyphenoxy)-2-propylalcohol as a raw material

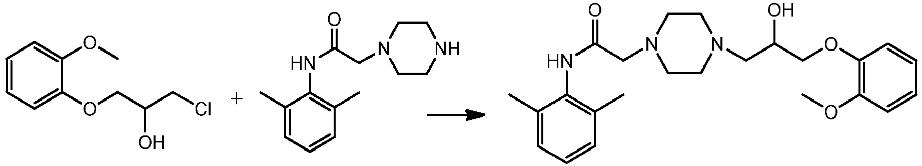

2.5 g (0.01 mol) of 1-chloro-3-(2-methoxyphenoxy)-2-propylalcohol, 3.4 g (0.013 mol) of N-(2,6-dimethylphenyl)-1-piperazinylacetamide, 3.2 g (0.03 mol) of sodium carbonate, 25 ml of isopropanol and 60 ml of toluene were successively added into a reaction flask and heated under reflux for 4.5 h till completion.

The former fraction whose main ingredient was isopropanol was collected by atmospheric distillation and then filtrated. The filtrate was washed with 3N HCl to get liquid having a pH of 1-2 and further treated with 50 ml of saturated sodium carbonate solution to adjust pH to 9-10. The product was extracted three times with 25 ml of dichloromethane each and the lower organic phase was combined. After the dichloromethane was removed by distillation under reduced pressure and rotary evaporation, the yellow viscous liquid was obtained and then further dissolved in about 15 ml of methonal. The tetrahydrofuran was then dropwise added under reflux till turbidity. The product was slowly crystallized with cooling and filtrated to get 3.16 g of white solid having a yield of 74% by vacuum drying at 40°C.

The result of ¹HNMR (CDCl₃) confirmed that the compound obtained is ranolazine. Purity by HPLC (area normalization method): 98.9%.

### 3.4: 1-bromo-3-(2-methoxyphenoxy)-2-propylalcohol as a raw material

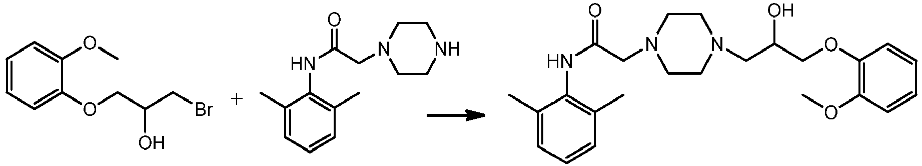

2.6 g (0.014 mol) of 1-bromo-3-(2-methoxyphenoxy)-2-propylalcohol, 3.1 g (0.012 mol) of N-(2,6-dimethylphenyl)-1-piperazinylacetamide, 5.5 g (0.04 mol) of potassium carbonate, 25 ml of ethanol and 50 ml of toluene were successively added into a reaction flask and heated under reflux for 3 h till completion.

The former fraction whose main ingredient was ethanol was collected by atmospheric distillation and then filtrated. The filtrate was washed with 3N HCl to get liquid having a pH of 1-2 and further treated with 50 ml of saturated sodium carbonate solution to adjust pH to 9-10. The product was extracted three times with 25 ml of dichloromethane each and the lower organic phase was combined. After the dichloromethane was removed by distillation under reduced pressure and rotary evaporation, the yellow viscous liquid was obtained and then further dissolved in about 10 ml of methonal. The tetrahydrofuran was then dropwise added under reflux till turbidity. The product was slowly crystallized with cooling and filtrated to get 3.73 g of white solid having a yield of 87.4% by vacuum drying at 40°C.

The result of ¹HNMR (CDCl₃) confirmed that the compound obtained is ranolazine. Purity by HPLC (area normalization method): 99.3%.

### 3.5: 1-bromo-3-(2-methoxyphenoxy)-2-propylalcohol as a raw material

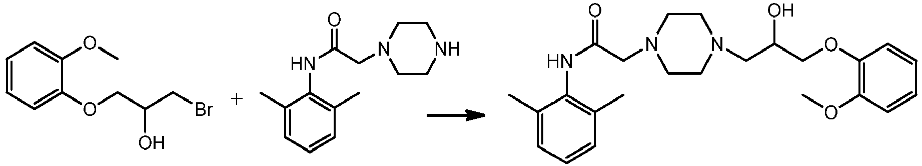

3.64 g (0.014 mol) of 1-bromo-3-(2-methoxyphenoxy)-2-propylalcohol, 3.1 g (0.012 mol) of N-(2,6-dimethylphenyl)-1-piperazinylacetamide, 4.3 g (0.04 mol) of sodium carbonate, 25 ml of methanol and 50 ml of toluene were successively added into a reaction flask and heated under reflux for 4 h till completion.

The former fraction whose main ingredient was methanol was collected by atmospheric distillation and then filtrated. The filtrate was washed with 3N HCl to get liquid having a pH of 1-2 and further treated with 50 ml of saturated sodium carbonate solution to adjust pH to 9-10. The product was extracted three times with 20 ml of dichloromethane each and the lower organic phase was combined. After the dichloromethane was removed by distillation under reduced pressure and rotary evaporation, the yellow viscous liquid was obtained and then further dissolved in about 10 ml of methonal. The tetrahydrofuran was then dropwise added under reflux till turbidity. The product was slowly crystallized with cooling and filtrated to get 3.4 g of white solid having a yield of 66% by vacuum drying at 40°C.

The result of ¹HNMR (CDCl₃) confirmed that the compound obtained is ranolazine. Purity by HPLC (area normalization method): 98.1%.

### 3.6: 1-bromo-3-(2-methoxyphenoxy)-2-propylalcohol as a raw material

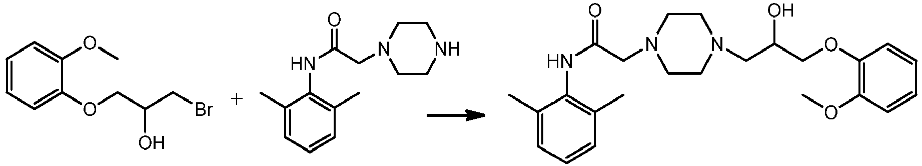

2.6 g (0.014 mol) of 1-bromo-3-(2-methoxyphenoxy)-2-propylalcohol, 3.1 g (0.012 mol) of N-(2,6-dimethylphenyl)-1-piperazinylacetamide, 5.5 g (0.04 mol) of potassium carbonate and 70 ml of toluene were successively added into a reaction flask and heated up to 80-85°C for 5 h till completion.

After cooling and filtering, the filtrate was washed with 3N HCl to get liquid having a pH of 1-2 and further treated with 50 ml of saturated sodium carbonate solution to adjust pH to 9-10. The product was extracted three times with 25 ml of dichloromethane each and the lower organic phase was combined. After the dichloromethane was removed by distillation under reduced pressure and rotary evaporation, the yellow viscous liquid was obtained and then further dissolved in about 10 ml of methonal. The tetrahydrofuran was then dropwise added under reflux till turbidity. The product was slowly crystallized with cooling and filtrated to get 2.6 g of white solid having a yield of 60.9% by vacuum drying at 40°C.

The result of ¹HNMR (CDCl₃) confirmed that the compound obtained is ranolazine. Purity by HPLC (area normalization method): 98.7%.

## Claims

1. A process for the preparation of ranolazine comprises the step of condensing N-(2,6-dimethylphenyl)-1-piperazinylacetamide with a compound of formula as given below to obtain ranolazine, in which X is chlorine or bromine.

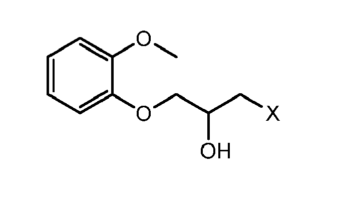

2. The process according to claim 1, wherein the condensation is carried out in the presence of alcohol, toluene or mixtures thereof and heated under reflux in an alkaline environment.

3. The process according to claim 1 or 2, wherein the mole ratio of N-(2,6-dimethylphenyl)-1-piperazinylacetamide to the compound of formula as given below is 0.8~1.3:1.

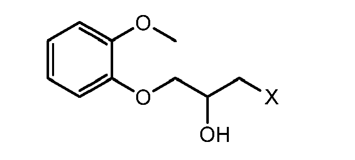

4. The process according to claim 1 or 2, wherein the condensation reaction is carried out for 3~5 h.

5. The process according to claim 2, wherein the alcohol is selected from methanol, ethanol, normal propyl alcohol and isopropyl alcohol.

6. The process according to claim 2, wherein the alkaline environment is formed by sodium carbonate, sodium bicarbonate, potassium carbonate, potassium bicarbonate or mixtures thereof.
